(19)

Europäisches
Patentamt
European
Patent Office
Office européen
des brevets

(11) **EP 1 811 304 A1**

(12) **EUROPEAN PATENT APPLICATION**

(43) Date of publication:
**25.07.2007 Bulletin 2007/30**

(51) Int Cl.:
*G01N 33/68* (2006.01)          *C07K 16/00* (2006.01)

(21) Application number: **06000969.3**

(22) Date of filing: **18.01.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **Friedrich-Alexander-Universität Erlangen-Nürnberg**
**91054 Erlangen (DE)**

(72) Inventors:
• **Wiltfang, Jens**
**91056 Erlangen (DE)**

• **Kornhuber, Johannes**
**90491 Nürnberg (DE)**
• **Henkel, Andreas Wolfram**
**91056 Erlangen (DE)**
• **Lewczuk, Piotr**
**91341 Röttenbach (DE)**
• **Klafki, Hans-Wolfgang**
**91052 Erlangen (DE)**

(74) Representative: **Rehberg Hüppe + Partner**
**Nikolausberger Weg 62**
**37073 Göttingen (DE)**

(54) **Large Aß-peptide binding particles (LAPS) in diagnosis and therapy of Alzheimer's dementia**

(57)     A method of analyzing a sample obtained from a patient for indications that the patient has or is susceptible to Alzheimer's dementia comprises the step of determining at least one concentration in the sample, the at least one concentration being a concentration of Aβ-peptide- and immunoglobulin-including particles.

EP 1 811 304 A1

**Description**

The present invention relates to a method of analyzing a sample obtained from a patient for indications that the patient has or is susceptible to Alzheimer's dementia, the method comprising the step of determining at least one concentration in the sample.

Further, the present invention relates to a method of producing an immunotherapy agent suitable for treating or preventing Alzheimer's dementia.

<u>Prior Art</u>

In view of novel therapies that may enter clinical trials in the near future, early and reliable diagnosis of Alzheimer's disease (AD) and other neurodegenerative diseases is gaining more and more importance. Any changes in biochemical composition of the brain metabolism should be predominantly reflected in the cerebrospinal fluid (CSF), because of its direct contact with the extracellular fluids of the central nervous system [1,2]. Therefore, determination of biomarkers in CSF, including beta-amyloid peptides (Aβ-peptides, in the following sometimes abbreviated as Aβ), tau and phospho-tau proved to be very important for differential diagnosis of dementias [3,4]. Commonly, Aβ-peptides are quantified by ELISA technique for AD diagnosis. Decrease of Aβ1-42 (in the following sometimes abbreviated as Aβ42) in CSF of AD patients was reported by many groups; however, this decrease is not AD specific but also observed in Lewy-body dementia, and Creutzfeldt-Jakob disease [5-7]. Precipitation of Aβ into amyloid plaques has been proposed as one potential mechanism for reduction of Aβ in CSF [8]; however, since CJD patients with decreased CSF Aβ1-42 levels do not have brain beta-amyloid plaques, other mechanisms than plaque-mediated precipitation may be responsible [5]. Such mechanisms could be high-avidity binding to carrier proteins, IgGs or formation of detergent-stable soluble oligomers, conditions which would result in decreased CSF concentrations of the monomeric fraction due to epitope masking [5]. There is evidence that Aβ-peptides exist in several structural conformations, one of these being the beta sheet structure [9]. This form which tends to have strong aggregation potency is considered to be the pathological form, occurring in AD-patients.

In 1998, Pitschke and colleagues reported [10] that exclusively CSF of AD patients, but not of controls, contained large amounts of large Aβ-peptide binding particles (LAPs). Detection of LAPs in CSF by confocal spectroscopy (ConSP) was therefore interpreted as a positive diagnosis for AD. This method was based on 'seeded polymerization' of fluorescent synthetic Aβ-peptides on preexisting Aβ aggregates and fibrils in CSF of AD patients. These kind of aggregates are supposedly the main component of amyloid plaques that are associated with Alzheimer's disease [11]. In the optical setup, the fluorescence of labeled molecules was detected as they entered a small illuminated confocal volume of approximately one femtoliter [12]. Large single aggregates deliver high intensive fluorescent flashes (peaks) when they pass through the confocal volume, making them easy to detect against the much lower background of monomers.

Recently, antibodies against Aβ peptides were found in CSF in AD patients but also in non-demented individuals [13]. Immune responses and auto-antibodies directed against aggregated forms of Aβ-peptides may not only be of diagnostic interest but may also have therapeutic potential. Schenk and coworkers reported striking effects of Aβ vaccination in transgenic mice [14] and subsequently, a clinical trial was launched [15].

A method of analyzing a sample obtained from a patient for indications that the patient has or is susceptible to Alzheimer's dementia, which comprises the step of determining at least one concentration in the sample, is known from EP 1 270 592 A1. Here, the concentrations of different Aβ-peptides are measured in a fraction of the sample which binds to a monoclonal antibody mbAb 1 E8 specific for Aβ1-x and Aβ2-x. The concentrations of the Aβ-peptides are measured using SDS-PAGE. Besides the concentrations of Aβ1-42 and Aβ1-40, a ratio of these concentrations denominated as Aβ42/40 is determined to discriminate the samples derived from AD patients from the samples derived from non AD patients. A suitable Aβ42/40 threshold value for this discrimination may be in the range of 0.09 to 0.11 but there is an overlap of the Aβ42/40 values of both groups.

<u>Problem of the Invention</u>

It is a problem of the present invention to provide a new and better discriminating method of analyzing a sample obtained from a patient for indications that the patient has or is susceptible to Alzheimer's dementia. It is a further problem of the present invention to provide a method of producing a new and promising immunotherapy agent suitable for treating or preventing Alzheimer's dementia.

<u>Solution of the Invention</u>

The problems of the present invention are solved by a method of analyzing a sample obtained from a patient having the features of independent claim 1, and by a method of producing an immunotherapy agent having the features of independent claim 9. Preferred embodiments of the first method are described in dependent claims 2 to 8. A preffered embodiment of the second new method is described in dependent claim 10.

## General Description of the Invention

Originally, the inventors of the present invention improved the detection sensitivity of the known ConSP setup by means of a microchannel flow-through about 20-fold to evaluate the potential of the detection of LAPs in CSF for clinical AD standard diagnostics. Surprisingly, the inventors found that LAPs are detected in both groups with high inter-individual variation, rendering this method useless for AD diagnosis.

To gain insight into molecular details of LAPs the inventors employed confocal microscopy for molecular imaging of LAPs, which revealed that LAPs are heterogeneous superaggregates that can be subdivided in at least 4 subtypes. Particularly, it is possible to identify a subtype of immunoglobulin (IgG) containing LAPs that binds very tightly to aggregated Aβ1-42 peptides (LAP-4 subtype), and that was detected in 42.1 % of all non AD controls but is virtually absent from CSF of AD patients.

These results not only offer improved early diagnosis of AD but also indicate an involvement of the immune system in regulating Aβ1-42 homeostasis. Thus, the present results are regarded as evidence that a potential Aβ1-42-clearing system involves immune complexes, patrols the CSF of some nAD controls, and is absent in all AD patients.

Based on these findings, the first new method of analyzing a sample obtained from a patient for indications that the patient has or is susceptible to Alzheimer's dementia comprises the step of determining a concentration of Aβ-peptide- and immunoglobulin-including particles or of Aβ-peptides or immunoglobulins bound in such Aβ-peptide- and immunoglobulin-including particles. Thus, the new method makes use of the fact that Aβ-peptides and IgGs seem to be bound in a different way in Aβ-peptide- and immunoglobulin-including particles in AD patients and in nAD patients. This difference in the binding properties can by quantified by the concentrations of Aβ-peptide- and immunoglobulin-including particles as well as by the concentrations of both the Aβ-peptides and the IgGs bound in these particles.

It is preferred to directly determine the at least one concentration in the first new method, but is also possible to indirectly determine each of the above stated concentrations in that, for example, the ratio of the Aβ-peptides and/or the IgGs not bound in Aβ-peptide- and immunoglubolin-including particles on the one hand and of the total Aβ-peptides and/or IgGs on the other hand is determined.

The data presented here have been obtained from cerebrospinalfluid as the sample obtained from AD and nAD patients. However, it is expected that similar data can also be obtained using other body fluids or body tissue homogenates as the sample obtained from the patient.

Preferably, the at least one concentration which is determined according to the first new method is a concentration of Aβ1-42-peptide- and immunoglobulin-including particles or a concentration of Aβ1-42-peptides in such Aβ-peptide- and immunoglobulin-including particles. I.e. the new method focuses on Aβ1-42 bound by IgGs in Aβ-peptide- and immunoglobulin-including particles. The particles including Aβ1-42 and IgGs also include Aβ1-40, the concentration of which may also be determined.

The concentration of Aβ1-42- and IgGg-including particles, which is determined according to the first new method, can be a concentration of large Aβ1-42-peptide-binding particles (LAPs). According to the findings of the inventors LAPs can be subdivided into at least four subtypes. Two of this subtypes which are denominated as subtype 3 and subtype 4 here include Aβ1-42 and IgGs. These two LAP-subtypes are of particular interest in the new method.

Particularly it is preferred, if the first new method concentrates on the determination of the concentration of LAPs of LAP-subtype 4. The LAPs of LAP-subtype 4 have a round shape in single molecule imaging, whereas LAPs of LAP-subtype 3 have a more ellipsoids shape in single molecule imaging. The LAPs of LAP-subtype 4 are not found in samples from AD patients. They are only found in some of the samples of non-AD patients. The nAD patients from which the samples including LAPs of LAP-subtype 4 have been taken may be regarded as not susceptible to Alzheimer's disease whereas the other nAD patients may have a susceptibility to AD. At least, the existence of LAPs of LAP-subtype 4 in a sample from a particular patient indicates that the patient even if having a dementia has no AD.

The LAPs of the different LAP-subtypes may be differentiated in image morphometry by well established parameters of area, brightness, shape and texture. The LAPs of LAP-subtypes 3 and 4 clearly only differ from each other with regard to the shape-parameter. Only the LAPs of LAP-subtype 4 show a shape-parameter of 0,7 to 1,0. The shape-parameter is calculated as $\left(\frac{4\Pi A}{P^2}\right)$ and varies from 0 to 1, the difference to 1 indicating the difference with regard to a round shape. The LAP-subtype having a round shape in single molecule imaging may further shows an area of 0.34 to 4.10 $\mu m^2$, a brightness-parameter of at least 15, and a texture-parameter of not more than 2.0 in image morphometry.

The results which are presented in detail in the following indicate that a general close correlation exists between LAPs and IgG concentration in CSF in controls but not in AD patients. Aβ-peptides (Aβ1-40 and Aβ1-42) copurified with immunoglobulins from CSF on beads which had been, for example, impregnated with protein A or protein G acting as an antibody binding the immunoglobulins.

The ratio Aβ42/40 in IgG-copurified Aβ-peptides discriminates AD patients and controls without overlap. Importantly, the discrimination is significantly improved as compared to direct Aβ1-40 and Aβ1-42 quantification in the same set of CSF samples by standard ELISA.

Thus, a more general method of analyzing a sample obtained from a patient for indications that the patient has or is susceptible to Alzheimer's dementia may be proposed, in which at least one concentration of immunoglobulin-bound or immunoglobulin-copurified Aβ-peptides, particularly of to Aβ1-40-peptides and Aβ1-42-peptides, is determined.

In one additional group of embodiments of this general method, a fraction binding to an immunoglobulin-binding substance is separated from the remainder of the sample. The substance binding IgGs can, for example, be Protein A or Protein G, both binding to IgGs of various kinds. A more specific antibody to selected IgGs may also be used.

In a particular embodiment of this group of embodiments, a ratio of the concentrations of Aβ1-42 and Aβ1-40 is determined in the separated fraction of the sample binding to the immunoglobulin-binding substance. It has been found that the value of this ratio is able to clearly differentiate between AD patients and nAD patients. A suitable upper threshold value for the ratio Aβ42/40 above which no AD patients are found may be set within a range of 0.12 to 0.20. Particularly the upper threshold value should be within a range of 0,125 to 0,130. This latter range is the observed gap between the values of AD patients and nAD patients.

For applying the more general method in an easy way, the assay used may comprise an immunoglobulin-binding substance for separating a fraction binding to this substance from the remainder of the sample, and two marker substances which selectively bind to Aβ1-40-peptides and Aβ1-42-peptides in the fraction of the sample bound to the immunoglobulin-binding substance and which allow for a determination of the concentrations of the Aβ1-40-peptides and of the Aβ1-42-peptides in the fraction of the sample. The immunoglobulin-binding substance may, for example be Protein A or Protein G. Suitable marker substances can be constructed according to known methods.

The new method of producing an immune therapy agent suitable for treating or preventing Alzheimer's dementia comprises the step of purifying an immunoglobulin from a subtype of immunoglobulin-including large Aβ1-42-peptide-binding particles (LAPs) having a round shape in single molecule imaging (LAP-subtype 4). It is expected that the IgGs included in the LAPs of LAP-subtype 4 have certain properties binding Aβ-peptides, particularly Aβ1-42, into these LAPs. Further, it is expected that the Aβ-peptides bound by these IgGs are no longer able to be involved in an outbreak of AD. This may have the reason that the LAPs of LAP-subtype 4 are cleared by an immune system.

Other features and advantages of the present invention will become apparent to one with skill in the art upon examination of the following figures and the detailed description.


**Brief Description of the Figures**


The invention can be better understood with reference to the attached figures.


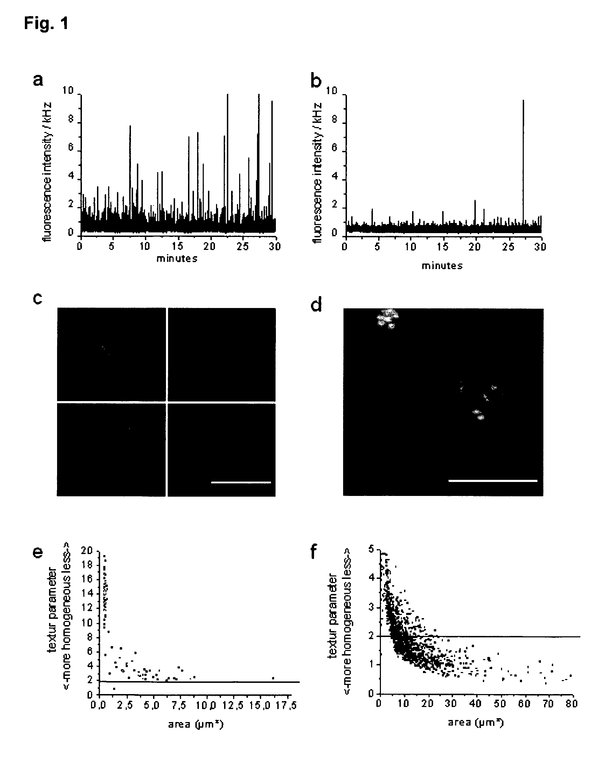

**Fig. 1** illustrates the identification of auto-aggregated Aβ1-42*.


a. Confocal spectroscopy: Fluorescence peaks resulting from LAPs in CSF of a nAD patient that bind Aβ1-42*.
b. peaks of Aβ1-42* in physiological buffer,
c. Confocal microscopy: Four examples of LAPs, formed from of auto-aggregated Aβ1-42* in physiological buffer (scale bar = 2 μm),
d. synthetic Aβ1-42, pre-aggregated for 24 h in physiological buffer, incubated with Aβ1-42* (scale bar = 5 μm),
e. LAP-area versus LAP-texture plot of auto-aggregated Aβ1-42* shows two groups of auto-Aβ1-42* aggregates,
f. low LAP-texture values discriminate auto-aggregates from other LAP types.


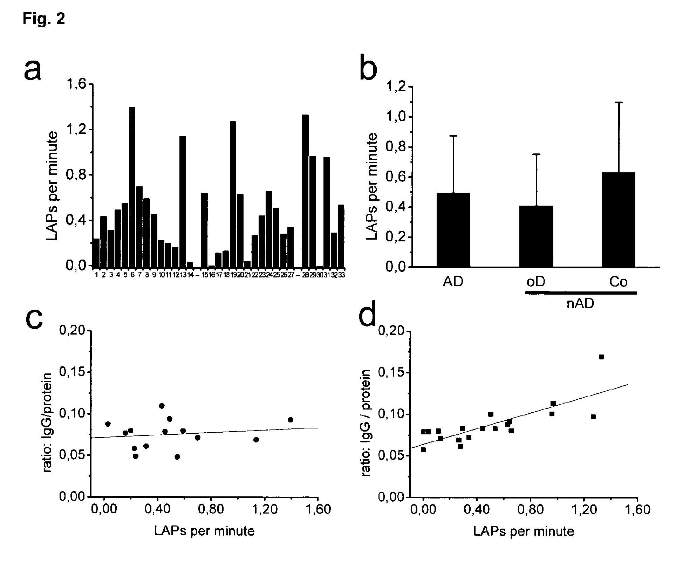

**Fig. 2** shows that LAP frequency is AD independent but correlates with IgG concentration:


a. Frequency of LAPs above 3000 kHz photon count in individual patients. ICD-10 diagnoses are specified: patients 1-14 comprise the patient group "AD", and patients 15-33 comprise "nAD", 1-10 = AD, 11-14 = AD of mixed type, 15 = dementia with aphasia, 16 = dementia with Parkinson's disease, 17-18 = vascular dementia, 19 = dementia, not specified, 20+21 = fronto-temporal dementia, 22-24 = Lewy-body dementia, 25-27 = Morbus Pick's disease, 28-32 = personality disorders, 33 = alcohol dependence;
b. Mean values ± standard deviation of 3 diagnostic groups show no significant differences at a threshold of 3000 kHz photon count;
c. Confocal spectroscopy: Linear correlation between LAP frequency and specific IgG concentration shows a highly significant correlation in nAD controls,
d. but no significant correlation in AD patients.

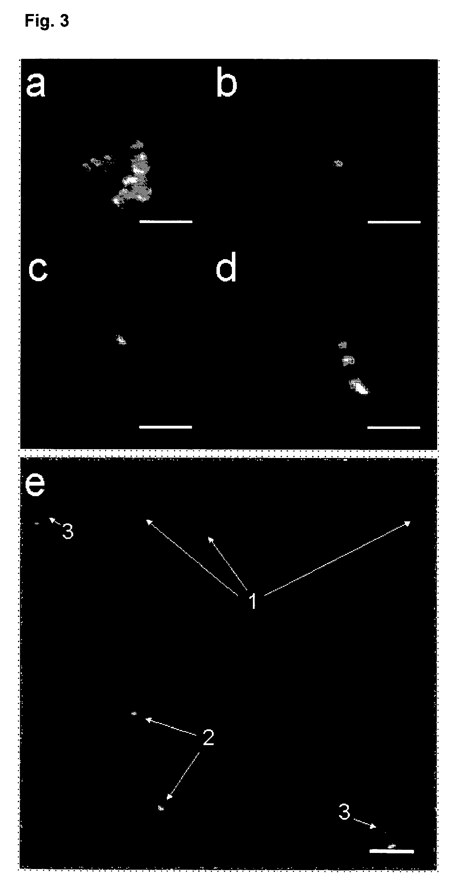

**Fig. 3** illustrates how confocal imaging discriminates four different LAP subtypes in CSF

a. Aβ1-42, 24 h pre-aggregated, labeled with Aβ1-42*;
b.; 1 % albumin in PBS forms aggregates with Aβ1-42*;
c. IgG-containing LAP-3 subtype;
d. IgG-containing LAP-4 subtype;
e. single confocal image, showing examples LAP subtypes: 1 = Aβ1-42* auto-aggregates, 2 = LAP-3 subtype, 3 = LAP-4 subtype
scale bars: a.- d. = 2 μm, e. =3.5 μm.

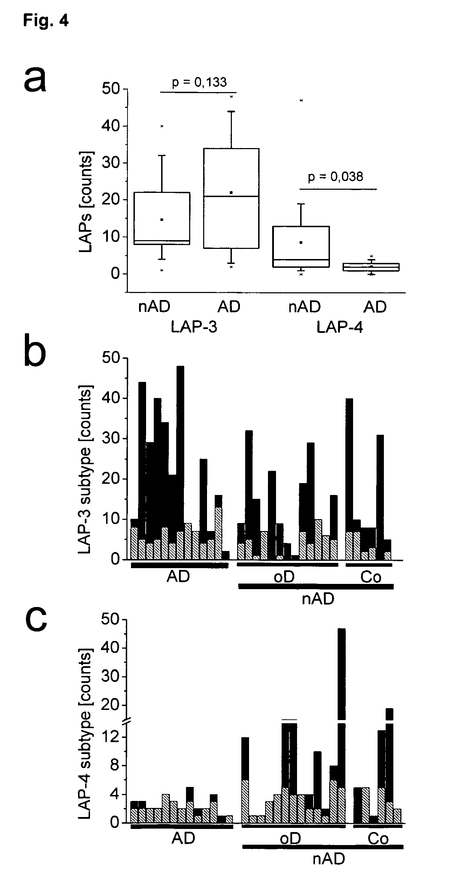

**Fig. 4** shows how protein A based depletion of IgG reduces LAPs:

a. Box plots show LAP-3 subtype frequency does not discriminate between diagnostic groups. LAP-4 subtype was detected only in a subgroup of nAD controls while AD patients had this subtype only within the background level;
b. Patient-specific changes in LAP subtype counts. LAP counts in individual patients before (solid bars), and after (shaded bars) protein A precipitation, (AD = Alzheimer's dementia, Co = non demented controls, oD = other dementias, nAD = Co and oD combined);
c. LAP-4 subtype is found in a subgroup of nAD controls only (solid bars). Protein A - precipitation (shaded bars) reduces LAP-4 subtype in these subjects.

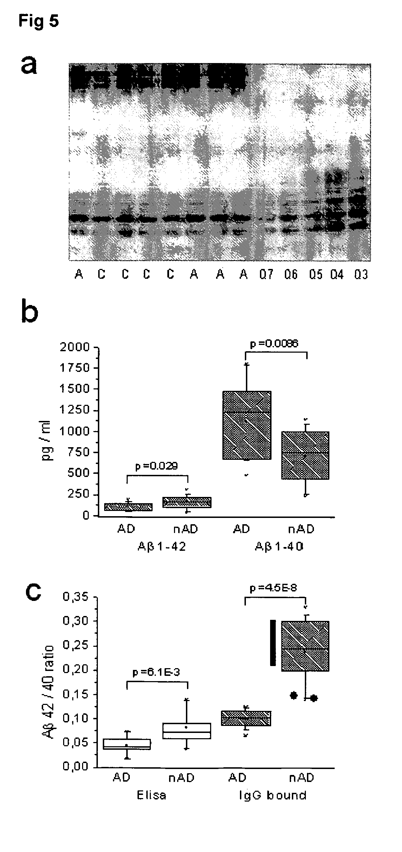

**Fig. 5** depicts how the protein A precipitation improves AD diagnosis based on Aβ42/40 ratio:

a. Urea-based Aβ-SDS-PAGE-immunoblot with antibody 1 E8 against Aβ peptides. A = AD patient, C = nAD control Q3 - Q7 = dilution series of a mixture of synthetic Aβ peptides;
b. Absolute concentrations of Aβ 1-42 and Aβ 1-40, co-precipitated with IgGs by protein A. Protein A beads bind IgG-Aβ 1-42 complexes significantly more effective in nAD controls while IgG-Aβ 1-40 complexes were significantly more precipitated in AD patients.
c. Elisa shows a highly significant separation between AD patients and nAD controls, directly measured in CSF, based on the Aβ42/40 ratio. Aβ-SDS-PAGE-immunoblot after protein A precipitation clearly improves separation between AD patients and nAD controls when Aβ42/40 ratio was calculated from IgG-bound Aβ peptides. Two Lewy-body dementia patients (⊗) have the lowest Aβ42/40 ratio within the nAD group. The ratio for one LBD-patient was not available. The black bar indicates the range of 5 controls without dementia.

## Detailed Description

### Results

Large Aβ-peptide binding particles in CSF are not specific for AD.

Synthetic Aβ-42 was fluorescently labeled with CY3 or FluorX (referred to Aβ1-42* in this application) and used for seeded aggregation studies. Analysis by confocal spectroscopy (ConSP) revealed that incubation of Aβ1-42* with CSF induced seeded aggregation of the probe on particles preexisting in CSF and produced many bright fluorescent peaks, as shown in figure 1 a, b.

ConSP and additionally confocal imaging (ConIM) were employed in order to analyze LAPs in CSF of AD patients and nAD controls. Confocal images of LAPs were analyzed by completely autonomic working computer algorithms which determined LAP-properties like area (size), brightness, shape and texture. The concentration of LAPs, determined by ConSP alone, varied considerably between individual patients but was not specific for AD.

Self-aggregation of fluorescent Aβ1-42 probe

The first problem encountered by using the ConSP setup was that the frequency of peaks with a brightness of less than 1000 kHz photon counts varied among different Aβ1-42* preparations, pointing to auto-aggregation of the fluorescent probe. Confocal imaging of Aβ1-42* auto-aggregates (figure 1c) showed that the majority consisted of small, irregular shaped structures with low brightness. In contrast, pre-aggregated synthetic Aβ1-42 which was not fluorescently labeled, formed large super-auto-aggregates *in vitro* that provided seeds for labeled Aβ1-42*. These structures were much larger than Aβ1-42* auto-aggregates which usually measured less than 2 μm, consisted of flat, punctuated structures that sometimes contained elongated tube-like fibrils. Figure 1 d gives an example of such structures that were also rather dim but nevertheless quite large. Self-aggregation of the Aβ1-42* probe could be greatly reduced by filtering it through a 0.22 μm membrane spin filter, right before usage in the experiment.

Auto-aggregates of Aβ1-42* could easily be identified by ConIM analysis because they were very inhomogeneous in texture. Figure 1e shows that virtually all Aβ1-42* auto-aggregates had texture values of more than 2, indicating a very heterogeneous brightness distribution, and were smaller than 4 μm² when texture was plotted against area. By contrast, when the fluorescent probe was mixed with CSF (figure 1f) of a patient or control person, many homogeneously bright LAPs became visible, which were clearly no auto-aggregates, because they had texture values of less than 2.

Frequency of LAPs varies among individual patients

In order to exclude Aβ1-42* auto-aggregates from ConSP analysis, only bright LAPs of more than 3000 kHz photon counts were included in the analysis, a value three times the threshold used by Pitschke and colleagues. Figure 2a illustrates that large individual differences in LAP frequency were found when patients were analyzed by ConSP.

Although there were striking and consistent inter-individual variations (triplicate determination) no significant differences (Kruskal-Wallis test, p=0.51) were found in LAP frequency between diagnostic groups, as shown in figure 2b. It was clearly apparent that CSF of some individual patients contained a large number of bright particles while others had virtually none that could surpass the applied strict criteria for LAP- brightness.

Close correlation between LAPs and immunoglobulins

In order to determine whether there was any correlation between LAPs, measured by ConSP and other CSF parameters, several neurochemical parameters were compared with the LAP frequency in individual patients (Table 1).

LAP frequency was significantly correlated to Aβ1-42 and tau only in nAD controls but the AD-specific CSF biomarker phospho-tau showed no correlation at all. Significant positive correlations were found between LAP frequency and immunoglobulin concentrations in nAD subjects only, while AD patients showed no correlation at all. In particular, the specific IgG concentration (IgG / protein) which corresponds to the relative contribution of IgG to total protein, was correlated (r=0.83; p<0.0001) to LAP frequency in nAD controls (figure 2c) but not in AD patients (figure 2d).

Individuals with normal or only minor elevated IgG concentrations of less than 50 mg/l in CSF had a significantly (p<0.0001) lower LAP frequency (10.4 ± 7.9) than patients with pathologically (more than 50 mg/l) high IgG (28.7 ± 10.7). In line with this result, the only patient in the nAD group, who was positive for oligoclonal bands, had the highest IgG concentration and also the highest LAP frequency.

Structural analysis of LAPs by confocal microscopy

To gain insight into structural details of LAPs, all samples were rescanned by the ConIM technique. LAPs were detected in all investigated CSF samples and in agreement with the results from ConSP, large inter-individual variations were observed in LAP frequency. ConIM allowed us to differentiate between auto-aggregates of Aβ1-42* and at least four structurally distinct LAP subtypes (figure 3). Auto-aggregates comprised 46% - 56 % of all detected fluorescent particles. They were clearly defined by heterogeneous texture, low brightness and small size (table 2).

LAP-1 subtype, rarely found in CSF, resembled structures that were observed when fluorescent Aβ1-42* was added to pre-aggregated synthetic, not labeled, Aβ1-42 seeds *in vitro.* The LAP-2 subtype consisted of probably protein-bound Aβ1-42* aggregates which were also small and heterogeneously textured but round shaped particles of variable brightness. It resembled particles generated in vitro by adding Aβ1-42* to physiological buffer, containing 1 % albumin as a seed.

The third and the fourth subtype comprised very bright LAPs that were either ellipsoid (LAP-3 subtype) or round-shaped (LAP-4 subtype). The fourth subtype did also comprise superaggregates that appeared as chains of large (~10 μm²) round pearls (figure 3d). Only a minute portion of these chains were expected to reveal this pearl chain like structure, since the LAPs were imaged by confocal microscopy. This technique retrieves image series, consisting of narrow frames of the scanned volume. The "chain of pearls" is not visible unless it is oriented exactly in the same orientation as the confocal layer. Therefore images of LAP-4 subtype were usually observed as large, bright and round spheres. Figure 4a, b shows that LAP-3 subtype was found in AD patients and nAD controls, while LAP-4 subtype was exclusively found in some nAD controls (figure 4a, c). AD patients had a LAP-4 frequency within the background level. Figure 4b shows that LAP-3 subtype was removed by protein A precipitation in the majority of individuals but there was no significant difference between AD and nAD groups. LAPs of subtype 4, only found in nAD controls, were also removed by protein A precipitation.

LAP-3, -4 contain Aβ1-42 binding IgGs

LAPs that were grouped into either the third or the fourth type resembled immune complexes, which are observed in autoimmune diseases. In order to test if LAP-3, -4 subtypes contained immunoglobulins, protein A precipitation of IgG and IgG-bound antigens were performed.

IgGs were almost completely removed from aliquots of CSF samples by protein A precipitation (data not shown). The IgG-depleted CSF was spiked with Aβ1-42* subsequently and ConIM images were taken. Both LAP-3, -4 subtype counts were substantially decreased in all individuals who had shown high LAP counts before IgG depletion (figure 4b, c).

IgG depletion improves Aβ1-42/1-40 ratio based AD diagnosis

Aβ40 and Aβ42 concentrations measured by Elisa before IgG depletion showed the well known decrease of Aβ-42 and virtually unchanged concentration of Aβ-40 in AD patients with respect to nAD controls. Moreover, tau and phospho-tau 181 were in the range as expected for AD patients, confirming that the available patients were a representative selection.

In order to determine if Aβ peptides were prominent antigens of protein A-bound IgG-antibodies, the fraction of co-precipitated Aβ peptides by urea-based SDS-PAGE/immunoblot were measured [16]. Figure 5a shows that indeed, the quintet of Aβ peptides (Aβ 1-37/38/39/40/42)[17] was detected in precipitated fractions, indicating the presence of Aβ-binding antibodies. Figure 5b shows that a significant (p = 0.029) decrease of Aβ 1-42 and surprisingly a significant (p = 0.009) increase of Aβ1-40 was found in the protein A bound IgG fraction in AD patients as compared to nAD controls. Figure 5c shows a highly significant p = 4.5 x 10^{-8}) 2.5 fold difference in the Aβ-42/40 ratios between AD patients and nAD controls that allowed clear separation of diagnostic groups without overlap. Noteworthy a subgroup of nAD controls without any cognitive deficits showed the highest values for the ratio of IgG-bound Aβ42/40, whereas two patients with Lewy body dementia were closest to the AD values (fig. 5c).

**Discussion**

Biomarkers in the CSF have become valuable tools in the early and reliable diagnosis of Alzheimer's disease and other dementias which is of importance to initiate suitable treatments. The levels of total tau protein, phospho-tau, and Aβ42 in CSF were shown to be of diagnostic value, in particular when evalutated in combination with each other[4]. Novel therapeutics for AD with strong disease modifying properties are expected to become available in the future, for review, see[18] and this will further emphasize the importance of accurate early diagnosis of AD and the predictive diagnosis of incipient AD in MCI patients.

In 1998, Pitschke and coworkers reported the detection of Aβ binding particles in CSF of AD patients by "seeded aggregation" of fluorescently labeled synthetic Aβ1-42* probes by confocal spectroscopy (ConSp). Importantly, these particles were claimed to exist exclusively in CSF from AD patients but not in samples from age matched controls, suggesting that they might be of diagnostic value. To the knowledge of the inventors, these highly promising findings have never been confirmed by other groups so far. Using an improved dynamic "flow through" setup, the detection probability for Aβ binding particles was substantially increased, and it was possible to detect 5-10 LAPs per minute, a frequency about 20 times higher than that reported in the original study. However, the exclusive confinement of LAPs to AD patients reported by Pitschke and colleagues could not be confirmed. Instead, large inter-individual variations with regard to the number of LAPs in CSF were observed independent of diagnosis. In order to exclude auto-aggregates of synthetic Aβ1-42* probes, the threshold was raised threefold over background level, but again virtually no difference in average LAP frequency between AD patients and nAD subjects was found. Thus, ConSP as such is not suitable for use in AD diagnostics.

Confocal microscopy for single molecule imaging (ConIM) was used to classify distinct subtypes of LAPs, based on brightness, area, shape and texture. Accordingly, the total population of CSF LAPs in all patients of the available collective was analyzed by fully automated quantitative computer-assisted morphometry. By this, four different LAP subtypes were clearly identified in CSF samples.

Images of LAP-3 and LAP-4 subtypes showed large multicentric supermolecular -structures, composed of multiple smaller core units which were secondarily attached to each other. These structures resembled immune complexes which have been observed in autoimmune diseases and in antigen-antibody aggregates generated *in-vitro* [19] LAP-3 and LAP-4 particles were substantially reduced after treatment of CSF samples with protein A coated magnetic beads, a finding that indicated the presence of immunoglobulins.

The elongated and relatively dim LAP-3 subtypes were not correlated to the diagnosis of the patients. LAP-4 subtypes were particularly interesting, because they were virtually absent in AD patients while they were detected in 42.1 % of all nAD controls.

Further support for the involvement of immunoglobulins in the composition of LAP-3 and LAP-4 subtypes came from the observation that LAP frequencies in CSF of nAD controls but not of AD patients, were positively correlated to immunoglobulin concentration. In addition significant correlations were observed between Aβ42, tau and LAPs in nAD controls. However, after Bonferroni correlation that accounts for multiple testing, only the the specific IgG/protein parameter remained significant. Several other CSF proteins and peptides did not show any significant correlations (table 1), suggesting a high specificity. Next, protein A precipitation was performed and the protein A bound fraction was analyzed by SDS-PAGE-immunoblot. At least 5 Aβ-peptides corresponding to Aβ (1-42, 1-40, 1-39, 1-38 and 1-37) were detected in this fraction and all of them have been observed as regularly constituents of CSF[17].

Taken together, these findings suggest that LAP-3 and LAP-4 probably contain auto-antibodies against Aβ-peptides with their bound antigens. Naturally occurring auto-antibodies directed against Aβ-peptides were detected preferentially in CSF of nAD controls and with significantly lower titers in CSF from AD patients, however without diagnostic value because of considerable overlap between diagnostic groups[13]. When the amounts of Aβ42 and Aβ40 peptides in the protein A enriched fraction were compared, it became obvious that the relative contributions of these C-terminal variants showed significant differences between nAD controls and AD patients. The observation that immunoglobulin bound Aβ42 was lower in AD patients as compared to controls, probably reflects the well known general decrease in Aβ42 in CSF which could also be confirmed in the available collective by standard ELISA. It is also possible that the increased amount of IgG-bound Aβ1-42 in nAD controls indicates the presence of auto-antibodies against this highly amyloidogenic species which may protect from AD onset. Recently, Hock and coworkers showed that the presence of auto-antibodies against Aβ1-42 were not correlated to the therapeutic benefit of active Aβ1-42 immunization, but a specific subfraction of auto-anti-antibodies against aggregated plaque-derived Aβ1-42 was[20]. Further evidence for a protective role of Ab42 auto-antibodies comes from a large, long-term epidemiological study, which demonstrated that these auto-antibodies were commonly detected in plasma of AD patients and age-matched healthy subjects. In those cases where auto-antibodies did not prevent the outbreak of AD, this was explained by low antibody titers and inadequate affinity and avidity of the antibodies to their antigens[21]. Moreover, immune senescence was also frequently observed in aged people[22].

Several studies, performed by Elisa, indicated no change in Aβ1-40 concentration in AD[23,24] Surprisingly, it was found that the amount of Aβ40 in the protein A enriched fraction was highly significant elevated in the AD patients. This observation supports the hypothesis that AD patients carry auto-antibodies against Aβ1-40 which do not protect them from AD. As a consequence from both observations, decreased Aβ42 plus increased Aβ40 in the immunoglobulin bound fraction, AD patients and nAD controls could be clearly discriminated without overlap when Aβ42/40 ratios were compared. This clear non-overlapping separation was found in spite of two LBD-patients included in the control group which are known to have also lower Aβ42/40 ratios[25]. Importantly, the discrimination was substantially better as compared to the discrimination on the basis of Aβ42/40 ratios calculated from standard ELISA tests.

In summary findings reported here provide evidence for a putative circulating immune (IgG)-based clearance system for soluble amyloidogenic proteins/peptides patrolling the central nervous system. A deficiency of this circulating IgG-clearance system in neurodegenerative disorders would result in increased tissue concentrations of soluble and finally precipitated amyloidogenic proteinaceous compounds but reduced concentrations of these amyloidogenic species within the clearing pool, e.g. IgG-bound Aβ1-42 in human body fluids. Future experiments might be directed towards a more detailed characterization of anti-Aβ auto-antibodies and the evaluation of their use for differential diagnosis of neurodegenerative disorders. Furthermore, the findings reported here are also be of interest in view of potential novel immunological therapeutic approaches for AD.

### Methods

#### Patients

The experiments were performed as double blind trials. CSF was obtained from all patients by a lumbar puncture. CSF was centrifuged (1600 x g, 15 min, RT), aliquoted, immediately frozen and stored at -80°C until assay. The patients (n=33) were diagnosed according to the criteria of ICD-10 and National Institute of Neurological and Communicative Disorders and Stroke-Alzheimer's Disease and Related Disorders Association (NINCDS-ADRDA)[26]. The diagnostic work-up included either cranial CT or predominantly MRI and routine blood assays. The extensive neuropsychological characterisation of the dementia patients followed the diagnostic criteria of the German Competence Net Dementias[27]. Only patients with early dementias (Mini Mental State Examination ≥ 20) were included in the study. The patients were divided into 3 subgroups: (1) AD (n=14): This group comprised patients with AD (n=8) and AD of mixed type (n=6); (2) oD (n=13): A group of patients with dementia of other origin, including Morbus Pick's disease (n=3), Lewy-body-dementia (n=3), and vascular dementia (n=4); (3) Co (n=6): Non-demented disease controls, comprising patients with heterogenous neurological or psychiatric diseases but without memory complaints. The patient groups (2) and (3) were summarized as "nAD" in the course of this article. CSF dementia markers like Aβ1-40, Aβ1-42, total Tau and phospho-Tau181 were measured in all patients. CSF aliquots for the analysis of IgG-bound Aβ-peptides were available in 9 of the AD patients, 13 of the oD patients and 5 of the Co patients. The results are presented in table 3.

Fluorescence labeling of synthetic Aβ1-42

The peptide was dissolved in water-free DMSO to a final concentration of 400 μM and centrifuged for 10 minutes at 14000 rpm at RT to remove large insoluble particles and kept on ice during the course of the experiments. 1 mg FluorX (Amersham Biosciences, Freiburg, Germany) was dissolved in a total volume of 1 ml reaction buffer (50 mM NaPO$_3$, pH=9.2, 0.2% SDS) and 20 μg of peptide (11 μl) was add to 100 μl FluorX solution and 9 μl reaction buffer for 30 min at RT. FluorX -labeled Aβ1-42 was used in all experiments on the confocal spectroscopy setup.

For confocal imaging of LAPs, 20 μg Aβ1-42 were labeled with Cy3 (Amersham Biosciences, Freiburg, Germany) according to manufacturer's instruction. Fluorescent Aβ1-42 is indicated as "Aβ1-42*" in this article.

The conjugated Aβ1-42* was separated from free fluorescent label by NAP5 column (Amersham Biosciences, Freiburg, Germany). The sample (120 μl) was allowed to sink into the gel bed and was eluted with 5 ml elution buffer (10 mM NaPO$_3$, pH 7.2, 0.2% SDS, 200 mM NaCl). The fluorescence in the fractions was determined by Fluorescence Correlation Spectroscopy (FCS) by mixing of 2 μl Aβ1-42* with 20 μl physiological buffer. 10 FCS curves were recorded for 1 minute each. By means of this technique, the free fluorescent molecules could be clearly discriminated from Aβ1-42*, based on different diffusion coefficients. Fractions containing exclusively conjugates were pooled and centrifuged through Micropore Ultrafree MC (BioRad) sterile spin filter (0.22 μm) for 5 minutes at 14000 RPM in order to remove auto-aggregates of Aβ1-42*.

'Seeded aggregation' assay in CSF with Aβ1-42*

CSF was collected according to the standardized guidelines for CSF-collection and storage of the German Competence Net Dementias [27], centrifuged (1600 g, 15 min, RT) and immediately frozen (-80°C), and it was used in all experiments. After thawing, 20 μl CSF was mixed with 2 μl of Aβ1-42* and allowed to bind to LAPs for 15-25 minutes at room temperature. In all confocal spectroscopy experiments, 20 μl were filled in the reservoir of the silicon elastomer microstructure which was connected to a microstructured channel. The confocal spot was centered 2-4 mm from the reservoir outlet of the channel in the middle of the channel. All experiments were performed as double blind trials in triplicate and baselines were normalized for analysis. The velocity of the sample (of $0.72 \pm 0.20$ mm/s) was controlled and recorded by FCS for each measurement [28]. All recorded data were normalized for different flow speeds by dividing the number of recorded fluorescent counts by the corresponding values from the FCS measurements.

Fast-flow confocal spectroscopy measurements

Fabrication of microstructures for fast-flow measurements was described in detail earlier [28,29]. In brief, the microstructures were cast from a silicone mold comprising the channel geometry and topology in the negative (upstanding) form. The silicon wafer was partly custom-made (GeSim, Großerkmannsdorf), coated with a thin 200-nm Teflon layer. The lengths of all microchannels were about 1.5 cm. Disposable microstructure channels could then easily be obtained by imprinting the complementary mold topology into silicon elastomer. In this routine, 2-3 ml of viscous elastomer kit (Sylgard 184, Dow Corning) was poured onto the silicon wafers and hardened on a heating plate for 30 min at 110 °C. The hardened silicon elastomer could then easily be peeled off the wafer, cut to the desired sizes, and punched at the end of the microchannels for filling-in of the Aβ-spiked CSF. Finally, it was sealed to a thin glass plate by manually pressing a coverslip of 170 μm thickness to the channel top, and mounted on an inverted microscope (Olympus IX 70).

The fluorescence from the flowing sample was detected in a self-made confocal setup. The 488 nm line of an Ar laser (Lasos, Jena, Germany) was adjusted into the microscope and focused by an objective (Olympus UplanApo 60 x, NA 1.2 W). Epifluorescence was collected by an optical fiber, and detected by an avalanche photodiode (SPCM CD3017, EG&G, Canada). Optical filters were introduced into the beam path (dichroic mirror 496 DCLP, and band pass filter 525 DF 50, both AHF, Tübingen, Germany). FCS was performed with the correlator card ALV-5000 (ALV Langen, Germany).

Confocal imaging and quantification of LAPs

20 μl CSF sample was spiked with Aβ1-42*, as described above and transferred onto a cover slip. The spiked CSF drop was scanned by a confocal laser microscope (LS 410, Leica, Wetzlar, Germany) in the 2-line scan average mode, using the 543 nm line of the argon laser. An immersion objective Fluotar 63 x, a long pass filter LP 570 was used, the zoom was set to 8 in all experiments and the pinhole was set to 1 airy. 50 image planes, separated by 1 μm in Z-direction, were collected at 2 different areas of the drop, resulting in 2 image stacks that measured 30 x 30 x 100 μm.

The image planes were processed and low-pass filtered with Metamorph® imaging software (Universal Imaging Co, USA). The 'integrated Morphology Analysis' routine from Metamorph® determined automatically the area, shape, brightness and texture. The algorithm for quantification in brief: LAPs were detected by thresholding the image to a sensitivity of 10 % above the average background. Detected objects were then masked and the brightness (Σgray value / # of pixels) was calculated. The brightness was proportional to the amount of Aβ1-42*, bound to the LAP. The area of an object represented the sum of all pixels in calibrated units. The shape factor $\left(\frac{4\Pi A}{P^2}\right)$, a value from 0 to 1, allowed to characterize an object as elongated = 0 or as a perfect circle = 1. The texture measured the uniformity of the gray levels in an object $\left(\sum_{i=1}^{N}\sum_{j=1}^{N}(i-j)^2 P_L\left(\frac{i}{j}\right)\right)$. Objects with uniform gray level have a texture difference moment (TDM) close to 0 while objects with greater variation of gray level have a larger TDM value. These four parameters were recorded for every detected LAP and subtypes of LAPs were characterized by setting specific parameter limits, examples were shown in table 2. These classifications were performed by a custom software ALDES, ver. 4.6.

Depletion of loG from CSF and analysis of loG-bound Aβ peptides

Magnetic microbeads (12.5 μL) pre-coated with protein A (Dynabeads® Protein A, Dynal, Germany) were transferred to a 1.5 ml reaction tube, briefly centrifuged and placed in a magnetic stand for one minute. The resulting supernatant was removed and 50 μL of CSF was added to the semi-dry beads. Samples were incubated at room temperature under rotation for 30 min, briefly centrifuged and magnetically collected. Bead-bound complexes were drawn to the side of the tube facing the magnet, and the CSF supernatant was removed with a pipette and used for further analysis. Beads were washed with 200 μl phosphate-buffered saline (PBS) / 0.1 % bovine serum albumin (BSA). For Aβ SDS-PAGE / immunoblot, bound complexes were eluted by heating the sample at 95 °C for 5 min with 25 μl of sample buffer (0.36 M bis-Tris, 0.16 M N,N-bis- (2-hydroxyethyl)-glycine, 1% (w/v) SDS, 15% (w/v) sucrose, and 0.004% (w/v) bromphenol blue) [17,30]. Urea-based SDS-PAGE/immunoblot for Aβ peptide analysis was performed as described [30]

**Quantification of routine CSF parameters and conventional neurochemical dementia markers**

CSF concentrations of immunoglobulins, albumin, and total CSF-protein were measured, according to manufacturer's instructions by means of the following commercially available assays: Albumin, IgG, IgA and IgM were quantified by nephelometry with the Immunochemistry System IMMAGE™ (Beckman-Coulter, Krefeld, Germany) and total protein quantification was performed with the Tina-quant™ assay (Roche/Hitachi, Basel, Switzerland).
The neurochemical dementia markers Aβ1-42 and Aβ1-40 were determined by ELISA from "The Genetics Company, Inc.", Schlieren, Switzerland and total Tau and phospho-Tau by ELISA from "Innogenetics", Gent, Belgium.

Data analysis and statistical calculation

Non-parametric statistical tests (rank correlation, Mann-Whitney-U, Kruskal - Wallis) were calculated with the statistical software package SPSS™ for Windows 12 (SPSS Inc., Chicago, II, USA). Additionally, the significance of the correlation coefficients was adjusted for multiple testing by the Bonferroni method (p-values multiplied with factor 17). All tests were two-sided. The level of significance was set at α=0.05.

**Tables**

**Table 1:** Linear correlation between LAP frequency and CSF parameters

| | nAD patients | | | AD patients | | |
|---|---|---|---|---|---|---|
| **Parameter** | | **r** | **p** | | **r** | **P** |
| Aβ1-42 | | 0.50 | 0.031* | | -0.21 | 0.464 |
| Aβ1-40 | | 0.43 | 0.073 | | -0.28 | 0.364 |
| Protein | | 0.29 | 0.223 | | -0.14 | 0.642 |
| Albumin | | 0.35 | 0.137 | | -0.16 | 0.578 |

(continued)

|  | nAD patients | | | AD patients | | |
| --- | --- | --- | --- | --- | --- | --- |
| Parameter |  | r | p |  | r | P |
| Tau |  | 0.53 | 0.021* |  | 0.09 | 0.765 |
| pTau |  | 0.07 | 0.794 |  | 0.07 | 0.830 |
| IgG |  | 0.56 | 0.012* |  | -0.13 | 0.670 |
| IgM |  | 0.48 | 0.037* |  | 0 | 0.988 |
| IgA |  | 0.60 | 0.007** |  | 0.06 | 0.850 |
| Aβ-42/1-40 |  | 0.27 | 0.283 |  | 0.02 | 0.957 |
| Aβ1-42/protein |  | 0.15 | 0.540 |  | -0.13 | 0.648 |
| Aβ1-40/protein |  | 0 | 0.987 |  | -0.10 | 0.748 |
| pTau/protein |  | -0.03 | 0.893 |  | 0.30 | 0.316 |
| IgG/protein |  | 0.83 | <0.001*** |  | 0.03 | 0.923 |
| IgG/albumin |  | 0.49 | 0.033* |  | 0.20 | 0.503 |
| IgM/protein |  | 0.43 | 0.069 |  | 0.15 | 0.615 |
| IgA/protein |  | 0.62 | 0.005** |  | 0.37 | 0.199 |
| r = correlation coefficient, p = probability, calculated by Mann-Whitney U-test; * = p < 0.05, ** = p < 0.01, *** = p < 0.001 | | | | | | |

**Table 2:** LAP definition by image-morphometry parameters

| LAP type | Area ($\mu m^2$) | Brightness (1-100) | Shape (0-1) | Texture (0-20) |
| --- | --- | --- | --- | --- |
| Aβ-auto-aggregates | 0.01 - 0.20 | 1 - 25 | 0.2 - 1.0 | 2 - 20 |
| Type 1: pre-aggregated Aβ | 3.40 - 6.80 | 1 - 25 | 0.0 - 0.5 | 0 - 4.5 |
| Type 2: protein/albumin | 0.07 - 0.34 | 5 - 25 | 0.5 - 1.0 | 2 - 4.5 |
| Type 3:IgG-Aβ1-42 | 0.34 - 4.10 | 2 - 100 | 0.1 - 0.7 | 0 - 1.3 |
| Type 4:IgG-Aβ1-42 | 0.34 - 4.10 | 15 - 100 | 0.7 - 1.0 | 0-2.0 |

**Table 3:** Clinical diagnostic parameters

| Parameter | mean | s.d. | p-value |
| --- | --- | --- | --- |
| AD Aβ1-40 | 8042,4 (pg/ml) | 2785,19 | 0.167 |
| nAD Aβ1-40 | 6705,1 (pg/ml) | 2037,29 | |
| nAD Aβ1-42 | 439,2 (pg/ml) | 211,14 | >0.001 |
| nAD Aβ1-42 | 733,4 (pg/ml) | 291,93 | |
| AD Aβ1-42/1-40 | 0,054 | 0,0237 | >0.001 |
| nAD Aβ 1-42/1-40 | 0,116 | 0,0466 | |
| AD Tau | 697,92 (pg/ml) | 334,85 | >0.001 |
| nAD Tau | 273,73 (pg/ml) | 203,55 | |
| AD pTau | 93,48 (pg/ml) | 40,07 | >0.001 |
| nAD pTau | 41,21 (pg/ml) | 22,72 | |

(continued)

| Parameter | mean | s.d. | p-value |
|---|---|---|---|
| AD age | 70,6 (years) | 6,55 | 0.012 |
| nAD age | 58,8 (years) | 15,21 | |

AD men: 6, AD women:8; nAD men: 10, nAD women: 9

## References

1. Lewczuk, P. et al. Tau protein phosphorylated at threonine 181 in CSF as a neurochemical biomarker in Alzheimer's disease: original data and review of the literature. Journal of Molecular Neuroscience 23, 115-122 (2004).

2. Kropp, S. et al. [Diagnostic steps in Alzheimer dementia before treatment with new antidimentives]. Fortschr Neurol Psychiatr 68, 257-61. (2000).

3. Verbeek, M.M., De Jong, D. & Kremer, H.P. Brain-specific proteins in cerebrospinal fluid for the diagnosis of neurodegenerative diseases. Ann Clin Biochem 40, 25-40 (2003).

4. Andreasen, N. & Blennow, K. CSF biomarkers for mild cognitive impairment and early Alzheimer's disease. Clin Neurol Neurosurg 107, 165-73 (2005).

5. Wiltfang, J. et al. Beta-amyloid peptides in cerebrospinal fluid of patients with Creutzfeldt-Jakob disease. Ann Neurol 54, 263-7 (2003).

6. Hulstaert, F. et al. Improved discrimination of AD patients using beta-amyloid(1-42) and tau levels in CSF. Neurology 52, 1555-62. (1999).

7. Otto, M. et al. Decreased beta-amyloidl-42 in cerebrospinal fluid of patients with Creutzfeldt-Jakob disease. Neurology 54, 1099-102. (2000).

8. Selkoe, D.J. Cell biology of the amyloid beta-protein precursor and the mechanism of Alzheimer's disease. Annu Rev Cell Biol 10, 373-403 (1994).

9. Antzutkin ON, B.J., Tycko R. Site-specific identification of non-beta-strand conformations in Alzheimer's beta-amyloid fibrils by solid-state NMR. Biophysical Journal 84, 3326-3335. (2003).

10. Pitschke, M., Prior, R., Haupt, M. & Riesner, D. Detection of single amyloid beta-protein aggregates in the cerebrospinal fluid of Alzheimer's patients by fluorescence correlation spectroscopy. Nat Med 4, 832-4. (1998).

11. Atwood, C.S., Martins, R.N., Smith, M.A. & Perry, G. Senile plaque composition and posttranslational modification of amyloid-beta peptide and associated proteins. Peptides 23, 1343-50 (2002).

12. Schwille, P., Bieschke, J. & Oehlenschlager, F. Kinetic investigations by fluorescence correlation spectroscopy: the analytical and diagnostic potential of diffusion studies. Biophys Chem 66, 211-28. (1997).

13. Du, Y. et al. Reduced levels of amyloid beta-peptide antibody in Alzheimer disease. Neurology 57, 801-5 (2001).

14. Schenk, D. et al. Immunization with amyloid-beta attenuates Alzheimer-disease-like pathology in the PDAPP mouse. Nature 400, 173-7 (1999).

15. Hock, C. et al. Generation of antibodies specific for beta-amyloid by vaccination of patients with Alzheimer disease. Nat Med 8, 1270-5 (2002).

16. Klafki, H.W., Wiltfang, J. & Staufenbiel, M. Electrophoretic separation of betaA4 peptides (1-40) and (1-42). Anal Biochem 237, 24-9. (1996).

17. Wiltfang, J. et al. Highly conserved and disease-specific patterns of carboxyterminally truncated Abeta peptides 1-37/38/39 in addition to 1-40/42 in Alzheimer's disease and in patients with chronic neuroinflammation. J Neurochem 81, 481-96. (2002).

18. Citron, M. Strategies for disease modification in Alzheimer's disease. Nat Rev Neurosci 5, 677-85 (2004).

19. Ljunghusen, O., Johansson, A. & Skogh, T. Hepatic immune complex elimination studied with FITC-labelled antigen. J Immunol Methods 128, 1-7 (1990).

20. Hock, C. et al. Antibodies against beta-amyloid slow cognitive decline in Alzheimer's disease. Neuron 38, 547-54 (2003).

21. Hyman, B.T. et al. Autoantibodies to amyloid-beta and Alzheimer's disease. Ann Neurol 49, 808-10 (2001).

22. Pawelec, G., Adibzadeh, M., Pohla, H. & Schaudt, K. Immunosenescence: ageing of the immune system. Immunol Today 16, 420-2 (1995).

23. Shoji, M. et al. Combination assay of CSF tau, A beta 1-40 and A beta 1-42(43) as a biochemical marker of Alzheimer's disease. J Neurol Sci 158, 134-40 (1998).

24. Mehta, P.D. et al. Plasma and cerebrospinal fluid levels of amyloid beta proteins 1-40 and 1-42 in Alzheimer disease. Arch Neurol 57, 100-5 (2000).

25. Lewczuk, P. et al. Neurochemical diagnosis of Alzheimer's dementia by CSF Abeta42, Abeta42/Abeta40 ratio and total tau. Neurobiol Aging 25, 273-81 (2004).

26. McKhann, G. et al. Clinical diagnosis of Alzheimer's disease: report of the NINCDS-ADRDA Work Group under the auspices of Department of Health and Human Services Task Force on Alzheimer's Disease. Neurology 34, 939-44. (1984).

27. KND. German Competence Nets Dementia, http://www.kompetenznetze de/navi/de/Kompetenznetze/demenzen.html. (2002).

28. Dittrich, P.S. & Schwille, P. An integrated microfluidic system for reaction, high-sensitivity detection, and sorting of fluorescent cells and particles. Anal Chem 75, 5767-74 (2003).

29. Dittrich, P.S. & Schwille, P. Spatial two-photon fluorescence cross-correlation spectroscopy for controlling molecular transport in microfluidic structures. Anal Chem 74, 4472-9 (2002).

30. Wiltfang, J. et al. Molecular biology of Alzheimer's dementia and its clinical relevance to early diagnosis and new therapeutic strategies. Gerontology 47, 65-71. (2001).

## Claims

1. A method of analyzing a sample obtained from a patient for indications that the patient has or is susceptible to Alzheimer's dementia, the method comprising the step of determining at least one concentration in the sample, **characterized in that** the at least one concentration is a concentration of Ap-peptide- and immunoglobulin-including particles or of Aβ-peptides or immunoglobulins bound in Aβ-peptide- and immunoglobulin-including particles.

2. The method of claim 1, wherein the sample includes cerebrospinal fluid.

3. The method of claim 1 or 2, wherein the at least one concentration is a concentration of Aβ1-42-peptide- and immunoglobulin-including particles.

4. The method of claim 3, wherein the concentration of Aβ1-42-peptide- and immunoglobulin-including particles is a concentration of immunoglobulin-including large Aβ1-42-peptide-binding particles (LAPs).

5. The method of claim 1 or 2, wherein the at least one concentration is a concentration of Aβ1-42-peptides in Aβ-peptide- and immunoglobulin-including particles.

6. The method of claim 3, wherein the concentration of Aβ1-42-peptides is a concentration of Aβ1-42-peptides in immunoglobulin-including large Aβ-peptide-binding particles (LAPs).

7. The method of claim 4, wherein the immunoglobulin-including LAPs belong to a LAP-subtype having a round shape in single molecule imaging, the round shape showing a shape-parameter in image morphometry of at least 0.7.

8. The method of claim 5, wherein the LAP-subtype having a round shape in single molecule imaging shows an area of 0.34 to 4.10 $\mu m^2$, a brightness-parameter of at least 15, and a texture-parameter of not more than 2.0 in image morphometry.

9. A method of producing an immune therapy agent suitable for treating or preventing Alzheimer's dementia, the method comprising the step of purifying an immunoglobulin from a subtype of immunoglobulin-including large Aβ1-42-peptide-binding particles (LAPs) having a round shape in single molecule imaging.

10. The method of claim 9, wherein the LAP-subtype shows an area of 0.34 to 4.10 $\mu m^2$, a brightness-parameter of at least 15, a shape-parameter of at least 0.7, and a texture-parameter of not more than 2.0 in image morphometry.

**Fig. 1**

Fig. 2

**Fig. 3**

Fig. 4

a

b

c

Fig 5

**European Patent**
**Office**

**EUROPEAN SEARCH REPORT**

Application Number

EP 06 00 0969

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TSUYOSHI ISHII ET AL: "IDENTIFICATION OF COMPONENTS OF IMMUNOGLOBULINS IN SENILE PLAQUES BY MEANS OF FLUORESCENT ANTIBODY TECHNIQUE" ACTA NEUROPATHOLOGICA, SPRINGER VERLAG, BERLIN, DE, vol. 32, no. 2, 11 August 1975 (1975-08-11), pages 157-162, XP009013619 ISSN: 0001-6322 * abstract * * page 158, lines 9-12; figure 1a * ----- | 1,2 | INV. G01N33/68 C07K16/00 |
| X | KAMETANI F ET AL: "A MONOCLONAL ANTIBODY HY20-54-16-3L TO LAMBDA (LAMBDA) LIGHT CHAIN OF HUMAN IMMUNOGLOBULIN REACTS WITH AMYLOID IN ALZHEIMER'S DISEASE BRAIN" NEUROSCIENCE LETTERS, LIMERICK, IE, vol. 117, no. 1/2, 4 September 1990 (1990-09-04), pages 62-67, XP009013620 ISSN: 0304-3940 * abstract; figure 3 * * page 65, lines 14-22 * * page 66, lines 9-11 * ----- -/-- | 1,2 | |
| | | | TECHNICAL FIELDS SEARCHED (IPC) G01N |

The present search report has been drawn up for all claims

| Place of search | Date of completion of the search | Examiner |
|---|---|---|
| The Hague | 9 June 2006 | Vadot-Van Geldre, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

**EUROPEAN SEARCH REPORT**

Application Number

EP 06 00 0969

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PARDRIDGE W M ET AL: "HIGH MOLECULAR WEIGHT ALZHEIMER'S DISEASE AMYLOID PEPTIDE IMMUNOREACTIVITY IN HUMAN SERUM AND CSF IS AN IMMUNOGLOBULIN G" BIOCHEMICAL AND BIOPHYSICAL RESEARCH COMMUNICATIONS, ACADEMIC PRESS INC. ORLANDO, FL, US, vol. 145, no. 1, 29 May 1987 (1987-05-29), pages 241-248, XP008019434 ISSN: 0006-291X * page 246, last paragraph - page 247, paragraph 1 * * abstract * | 1,2 | |
| X | ----- EIKELENBOOM P ET AL: "IMMUNOGLOBULINS AND COMPLEMENT FACTORS IN SENILE PLAQUES AN IMMUNOPEROXIDASE STUDY" ACTA NEUROPATHOLOGICA, SPRINGER VERLAG, BERLIN, DE, vol. 57, no. 2/3, 1982, pages 239-242, XP008019447 ISSN: 0001-6322 * abstract * * page 239, column 1 * * page 240, column 1, last paragraph - column 2, paragraph 1 * ----- -/-- | 1,2 | TECHNICAL FIELDS SEARCHED (IPC) |

The present search report has been drawn up for all claims

| Place of search | Date of completion of the search | Examiner |
|---|---|---|
| The Hague | 9 June 2006 | Vadot-Van Geldre, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

# EUROPEAN SEARCH REPORT

Application Number

EP 06 00 0969

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,A | PITSCHKE M ET AL: "DETECTION OF SINGLE AMYLOID BETA-PROTEIN AGGREGATES IN THE CEREBROSPINAL FLUID OF ALZHEIMER'S PATIENTS BY FLUORESCENCE CORRELATION SPECTROSCOPY"<br>NATURE MEDICINE, NATURE PUBLISHING GROUP, NEW YORK, NY, US,<br>vol. 4, no. 7, July 1998 (1998-07), pages 832-834, XP001073409<br>ISSN: 1078-8956<br>* page 833, column 2, paragraph 3; figures 2,3 *<br>----- | 1-10 | |
| D,A | US 2003/166019 A1 (WILTFANG JENS ET AL) 4 September 2003 (2003-09-04)<br>* paragraphs [0014], [0018], [0025]; figures 4,5,12 *<br>----- | 1-10 | |
| A | GEYLIS V ET AL: "Human monoclonal antibodies against amyloid-beta from healthy adults"<br>NEUROBIOLOGY OF AGING, TARRYTOWN, NY, US,<br>vol. 26, no. 5, May 2005 (2005-05), pages 597-606, XP004748495<br>ISSN: 0197-4580<br>* page 599, paragraph 3.2 *<br>----- | 9,10 | TECHNICAL FIELDS SEARCHED (IPC) |

The present search report has been drawn up for all claims

| Place of search | Date of completion of the search | Examiner |
|---|---|---|
| The Hague | 9 June 2006 | Vadot-Van Geldre, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

    .......................................................................
& : member of the same patent family, corresponding document

EPO FORM 1503 03.82 (P04C01)

**EP 1 811 304 A1**

## ANNEX TO THE EUROPEAN SEARCH REPORT
## ON EUROPEAN PATENT APPLICATION NO.

EP 06 00 0969

09-06-2006

| Patent document cited in search report | Publication date | Patent family member(s) | Publication date |
|---|---|---|---|
| US 2003166019 A1 | 04-09-2003 | AT 277952 T<br>DE 50103881 D1<br>EP 1270592 A1<br>ES 2228697 T3<br>JP 2003012700 A | 15-10-2004<br>04-11-2004<br>02-01-2003<br>16-04-2005<br>15-01-2003 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1270592 A1

**Non-patent literature cited in the description**

- **LEWCZUK, P. et al.** Tau protein phosphorylated at threonine 181 in CSF as a neurochemical biomarker in Alzheimer's disease: original data and review of the literature. *Journal of Molecular Neuroscience,* 2004, vol. 23, 115-122
- **KROPP, S. et al.** Diagnostic steps in Alzheimer dementia before treatment with new antidimentives. *Fortschr Neurol Psychiatr,* 2000, vol. 68, 257-61
- **VERBEEK, M.M. ; DE JONG, D ; KREMER, H.P.** Brain-specific proteins in cerebrospinal fluid for the diagnosis of neurodegenerative diseases. *Ann Clin Biochem,* 2003, vol. 40, 25-40
- **ANDREASEN, N. ; BLENNOW, K.** CSF biomarkers for mild cognitive impairment and early Alzheimer's disease. *Clin Neurol Neurosurg,* 2005, vol. 107, 165-73
- **WILTFANG, J. et al.** Beta-amyloid peptides in cerebrospinal fluid of patients with Creutzfeldt-Jakob disease. *Ann Neurol,* 2003, vol. 54, 263-7
- **HULSTAERT, F. et al.** Improved discrimination of AD patients using beta-amyloid(1-42) and tau levels in CSF. *Neurology,* 1999, vol. 52, 1555-62
- **OTTO, M. et al.** Decreased beta-amyloidl-42 in cerebrospinal fluid of patients with Creutzfeldt-Jakob disease. *Neurology,* 2000, vol. 54, 1099-102
- **SELKOE, D.** J. Cell biology of the amyloid beta-protein precursor and the mechanism of Alzheimer's disease. *Annu Rev Cell Biol,* 1994, vol. 10, 373-403
- **ANTZUTKIN ON, B.J. ; TYCKO R.** Site-specific identification of non-beta-strand conformations in Alzheimer's beta-amyloid fibrils by solid-state NMR. *Biophysical Journal,* 2003, vol. 84, 3326-3335
- **PITSCHKE, M. ; PRIOR, R. ; HAUPT, M. ; RIESNER, D.** Detection of single amyloid beta-protein aggregates in the cerebrospinal fluid of Alzheimer's patients by fluorescence correlation spectroscopy. *Nat Med,* 1998, vol. 4, 832-4
- **ATWOOD, C.S. ; MARTINS, R.N. ; SMITH, M.A. ; PERRY, G.** Senile plaque composition and posttranslational modification of amyloid-beta peptide and associated proteins. *Peptides,* 2002, vol. 23, 1343-50
- **SCHWILLE, P. ; BIESCHKE, J. ; OEHLEN-SCHLAGER, F.** Kinetic investigations by fluorescence correlation spectroscopy: the analytical and diagnostic potential of diffusion studies. *Biophys Chem,* 1997, vol. 66, 211-28
- **DU, Y. et al.** Reduced levels of amyloid beta-peptide antibody in Alzheimer disease. *Neurology,* 2001, vol. 57, 801-5
- **SCHENK, D. et al.** Immunization with amyloid-beta attenuates Alzheimer-disease-like pathology in the PDAPP mouse. *Nature,* 1999, vol. 400, 173-7
- **HOCK, C. et al.** Generation of antibodies specific for beta-amyloid by vaccination of patients with Alzheimer disease. *Nat Med,* 2002, vol. 8, 1270-5
- **KLAFKI, H.W. ; WILTFANG, J. ; STAUFENBIEL, M.** Electrophoretic separation of betaA4 peptides (1-40) and (1-42. *Anal Biochem,* 1996, vol. 237, 24-9
- **WILTFANG, J. et al.** Highly conserved and disease-specific patterns of carboxyterminally truncated Abeta peptides 1-37/38/39 in addition to 1-40/42 in Alzheimer's disease and in patients with chronic neuroinflammation. *J Neurochem,* 2002, vol. 81, 481-96
- **CITRON, M.** Strategies for disease modification in Alzheimer's disease. *Nat Rev Neurosci,* 2004, vol. 5, 677-85
- **LJUNGHUSEN, O. ; JOHANSSON, A. ; SKOGH, T.** Hepatic immune complex elimination studied with FITC-labelled antigen. *J Immunol Methods,* 1990, vol. 128, 1-7
- **HOCK, C. et al.** Antibodies against beta-amyloid slow cognitive decline in Alzheimer's disease. *Neuron,* 2003, vol. 38, 547-54
- **HYMAN, B.T. et al.** Autoantibodies to amyloid-beta and Alzheimer's disease. *Ann Neurol,* 2001, vol. 49, 808-10
- **PAWELEC, G. ; ADIBZADEH, M. ; POHLA, H. ; SCHAUDT, K.** Immunosenescence: ageing of the immune system. *Immunol Today,* 1995, vol. 16, 420-2

- **SHOJI, M. et al.** Combination assay of CSF tau, A beta 1-40 and A beta 1-42(43) as a biochemical marker of Alzheimer's disease. *J Neurol Sci,* 1998, vol. 158, 134-40
- **MEHTA, P.D. et al.** Plasma and cerebrospinal fluid levels of amyloid beta proteins 1-40 and 1-42 in Alzheimer disease. *Arch Neurol,* 2000, vol. 57, 100-5
- **LEWCZUK, P. et al.** Neurochemical diagnosis of Alzheimer's dementia by CSF Abeta42, Abeta42/Abeta40 ratio and total tau. *Neurobiol Aging,* 2004, vol. 25, 273-81
- **MCKHANN, G. et al.** Clinical diagnosis of Alzheimer's disease: report of the NINCDS-ADRDA Work Group under the auspices of Department of Health and Human Services Task Force on Alzheimer's Disease. *Neurology,* 1984, vol. 34, 939-44
- **DITTRICH, P.S. ; SCHWILLE, P.** An integrated microfluidic system for reaction, high-sensitivity detection, and sorting of fluorescent cells and particles. *Anal Chem,* 2003, vol. 75, 5767-74
- **DITTRICH, P.S. ; SCHWILLE, P.** Spatial two-photon fluorescence cross-correlation spectroscopy for controlling molecular transport in microfluidic structures. *Anal Chem,* 2002, vol. 74, 4472-9
- **WILTFANG, J. et al.** Molecular biology of Alzheimer's dementia and its clinical relevance to early diagnosis and new therapeutic strategies. *Gerontology,* 2001, vol. 47, 65-71